# EUROPEAN PATENT APPLICATION

(11) **EP 4 516 264 A1**
(43) Date of publication of application: **05.03.2025**
(21) Application number: 23795664.4
(22) Date of filing: 28.04.2023
(51) Int. Cl.: A61F 2/24

(54) **DELIVERY SYSTEM FOR INTERVENTIONAL HEART VALVE STENT**

(30) Priority: 29.04.2022 CN 202210474024; 27.07.2022 CN 202210896187; 31.03.2023 CN 202310358575; 26.04.2023 CN 202310500068
(71) Applicant: Wuhan Weike Medical Technology Co. Ltd, Wuhan, Hubei 430000 (CN)
(72) Inventor: CHEN, Song, East Lake New Technology Development Zone Wuhan, Hubei 430000 (CN); WANG, Xueli, East Lake New Technology Development Zone Wuhan, Hubei 430000 (CN); ZHANG, Changdong, East Lake New Technology Development Zone Wuhan, Hubei 430000 (CN); SUN, Ming, East Lake New Technology Development Zone Wuhan, Hubei 430000 (CN); WU, Chunlin, East Lake New Technology Development Zone Wuhan, Hubei 430000 (CN)
(74) Representative: Murgitroyd & Company
(86) International application number: PCT/CN2023/091868
(87) International publication number: WO 2023/208232

(57) **Abstract**

A delivery system for an interventional heart valve stent (200) is detachably connected to two ends of a heart valve stent (200) by means of a stent securing assembly; the axial movement of a support tube (12) along an inner core (11) allows the heart valve stent (200) to enter and exit from a capsule chamber (23) or from an accommodating cavity, thus achieving a bidirectional detachable connection method, which, compared with a unilateral securing method, allows the release position of the heart valve stent (200) to be more precise, thereby avoiding excess adjustment work.

## Description

### TECHNICAL FIELD

The present application relates to the technical field of interventional heart valve surgery related equipment, and in particular, to a delivery system for an interventional heart valve stent.

### BACKGROUND

With the aging of the population, incidence of valvular heart diseases has increased significantly. Currently, traditional surgical treatment is still preferred treatment for most patients with severe valvular diseases, but there are high risks of trauma, postoperative mortality, and complications. In recent years, transcatheter valve implantation/repair has gradually matured and been widely used, especially transcatheter aortic valve implantation (TAVR/TAVI), which has sufficient evidence-based evidence and greatly reduced trauma, has been recommended by European and American guidelines for treatment of heart valve diseases, and is a milestone in the field of interventional therapy for heart valve diseases.

The TAVI is a new technology that uses an interventional method to implant artificial aortic valves, which was first reported and used by Dr. Criber in France in 2002, brings hope for treatment of patients with severe aortic stenosis (AS) who have lost an opportunity for surgery (e.g. >80 years old), and opens a new page in history of interventional cardiovascular therapy. In the following 10 years, with improvement of devices and accumulation of experience, TAVI technology has been continuously improved and has been carried out in more than 230 heart centers in nearly 40 countries, with a total number of operations exceeding 150,000. Especially after a series of registration studies and randomized controlled studies have successively confirmed its effectiveness, feasibility, and safety, the TAVI technology has become a preferred treatment method for severe AS patients who cannot undergo surgical valve replacement. Clinically, artificial biological valves used in the TAVI mainly include the following two types: balloon-expandable Edwards Sapien (Edwards Corporation) and self-expanding CoreValve (Medtronic Inc.). The TAVI technology has made remarkable progress internationally and has been initially applied in China, with equally broad prospects. Currently, the domestic market of heart valve devices is highly monopolized by foreign brands, and foreign-funded companies such as EdwardsLifesciences, Medtronic, LivaNova (acquired by Solin), St. Jude (acquired by Abbott) and On-X occupy about 85% of the market share. EdwardsLifesciences and Medtronic have a full product pipeline from mechanical valves, biological valves to transcatheter interventional valves, and a number of domestic device companies have emerged in China. Currently, three domestic transcatheter aortic valves have been approved by Chinese CFDA for marketing, namely Venus-A from Qiming Medical, J-valve from Suzhou Jiecheng, and VitaFlow from Microinvasive Heart Tong, but none of the domestic companies have an absolute leading advantage.

According to statistical analysis of an echocardiogram database of hospital patients, in patients aged 65 to 74 years (49,995 cases) and ≥75 years (34,671 cases), detection rates of moderate or severe aortic insufficiency (AR) were 2.12% and 2.85%, respectively, detection rates of moderate or severe aortic stenosis (AS) were 0.75% and 0.89%, respectively, and detection rates of severe aortic insufficiency (SAR) and severe aortic stenosis (SAS) were 0.52% vs 0.95% and 0.54% vs 0.57%, respectively. As can be seen, the elderly in China are more inclined to aortic insufficiency in aortic valve degeneration. There are some differences between patients with aortic valve diseases in China and Western countries: ① The proportion of bicuspid aortic valves in China is relatively high, the proportion of patients with bicuspid aortic valves is 40% to 23%, which is much higher than 1.6% to 9.3% in Western countries, and the bicuspid aortic valve is listed as an exclusion criterion in a large number of large-scale TAVR clinical studies in Western countries. (2) A degree of aortic valve calcification is higher in Chinese patients. ③ Aortic regurgitation is more than aortic stenosis in China. ④ An inner diameter of a femoral artery is relatively thin, and an average inner diameter of the femoral artery in TAVR candidate cases in China is 6.5 mm.

Through a delivery system, an artificial valve stent can be delivered to the aortic valve and opened to complete implantation of the artificial valve and restore a valve function. There are still many problems affecting accurate release of the artificial valve stent during mounting of the delivery system and the artificial valve stent.

### SUMMARY

Based on the above, the present application provides a delivery device for an interventional heart valve stent to satisfy accommodation and release of a heart valve stent.

A delivery system for an interventional heart valve stent includes:
a delivery tube assembly including an inner core, a support tube, an inner sheath tube, and an outer sheath tube that are sequentially and movably sleeved from inside to outside, wherein two ends of the inner core extend out of two ends of the support tube, respectively, and a distal end of the inner sheath tube is provided with an accommodating cavity; and
a stent securing assembly capable of being detachably connected to two ends of a heart valve stent, the stent securing assembly including a capsule chamber, wherein a distal end of the inner core extends into the capsule chamber from an opening of a proximal end of the capsule chamber and is fixedly connected to the capsule chamber, and the support tube is capable of driving the heart valve stent to move axially to enablew the heart valve stent to move in and out of the capsule chamber or from the accommodating cavity. The technical solutions of the present application to solve the above technical problems are as follows.

The technical solution of the present application to solve the above technical problem is as follows.

The delivery system for an interventional heart valve stent according to embodiments of the present application is detachably connected to two ends of the heart valve stent through the stent securing assembly. The heart valve stent is enabled to move in or out of the capsule chamber or the accommodating cavity through an axial movement of the support tube along the inner core, thus achieving a bidirectional detachable connection method. Compared with a unilateral fixing method, a release position of the heart valve stent is more accurate, thereby avoiding unnecessary adjustment work.

On the basis of the above technical solution, the present application may further make the following improvement.

Further, the stent securing assembly further includes a securing member, a securing block, and a limiting member;
the securing member includes a support arm connected to the support tube and extending towards a distal end, the securing block is connected to a distal end of the support tube, and the support arm and the securing block are respectively configured to be detachably connected to a proximal end and a distal end of the heart valve stent; and
the limiting member has an elongated strip structure, the limiting member axially extend through the support tube and is capable of extending out of the two ends of the support tube, and a distal end of the limiting member is configured to connect and limit the proximal end of the heart valve stent and the support arm, so as to prevent the proximal end of the heart valve stent and the support arm from being separated from each other.

Further, a diameter of an opening of the capsule chamber is capable of being radially expanded, the support tube is provided with a guide member adjacent to the distal end thereof, and the guide member is capable of guiding the capsule chamber to move into the outer sheath tube.

Further, the guide member includes a connecting portion and a plurality of guide arms, the connecting portion is fixedly connected to an outer wall of the support tube, and the plurality of guide arms extend towards a distal end and elastically expand outwards.

Further, a distal end of the guide arm includes a transition portion, the transition portion is bent towards a side adjacent to an axis of the support tube relative to the guide arm.

Further, the capsule chamber includes a chamber body and a barrier opening portion, the chamber body is of a straight cylindrical structure, the barrier opening portion is of in a straight cylindrical shape without an external force and is capable of being expanded into a bell-mouth shape under an external force, and an inner side of the guide arm is capable of elastically pressing the barrier opening portion.

Further, the barrier opening portion is provided with a plurality of axial notches, the plurality of axial notches are evenly distributed around a circumferential direction of the capsule chamber, and a portion between adjacent axial notches form a barrier strip.

Further, a side surface of the chamber body is provided with a plurality of curved notches with a same length, the plurality of curved notches are distributed along an axial direction of the chamber body, adjacent curved notches are staggered, and portions between gaps between two ends of curved notches are connected to form a connecting rib that is spiral along the axial direction.

Further, the delivery system for an interventional heart valve stent further includes a control handle and two bending adjustment handles, the control handle is connected to a proximal end of the support tube and is capable of driving the support tube to move axially, the control handle includes a first stroke assembly configured to control an axial movement distance of the support tube, the two bending adjustment handles are provided at a proximal end of the inner sheath tube and a proximal end of the outer sheath tube, respectively, corresponding distal ends of the inner sheath tube and the outer sheath tube are bent in a same plane or different planes through a traction wire, and the bending adjustment handles each includes a second stroke assembly configured to control a bending curvature of the corresponding inner sheath tube or the outer sheath tube.

Further, the control handle is arranged along an axial direction of the support tube, the two bending adjustment handles are sequentially arranged on a side of the control handle adjacent to a distal end of the support tube, the bending adjustment handle configured to adjust the inner sheath tube is arranged closer to the control handle, the bending adjustment handle includes an axial portion and a branch portion, the axial portion is arranged along the axial direction of the support tube, the proximal ends of the inner sheath tube and the outer sheath tube are fixed to the axial portions of the corresponding bending adjustment handles, the branch portion forms an acute angle with the axial portion, and the second stroke assembly is arranged on the branch portion.

Further, the control handle includes a handle main body sleeve, a control sleeve, and a control sliding block, the first stroke assembly includes a first stroke sleeve and a first stroke indicating member, the handle main body sleeve is provided with an axial control slideway, an inner side of the control sleeve drives the control sliding block to slide along the axial control slideway through threaded fit, the support tube is fixed to a distal end of the handle main body sleeve, the support tube extends into the distal end of the handle main body sleeve and is fixed to the control sliding block, the first stroke sleeve is fixedly sleeved on an outer side of the control sleeve, the first stroke sleeve is provided with a first stroke slideway arranged along an axial direction thereof, and the outer side of the control sleeve drives the first stroke indicating member to slide along the first stroke slideway through threaded fit.

Further, the control handle further includes a dismounting member detachably mounted at a proximal end of the handle main body sleeve and connected to a proximal end of the limiting member, and when the heart valve stent is located in the capsule chamber, the limiting member is pulled by the dismounting member, so that a distal end of the limiting member releases a connection and limitation between a proximal end of the heart valve stent and the support arm.

Further, the axial portion includes an axial sleeve, a hemostatic valve, and a proximal fixing cover, the axial sleeve is provided with an axial through hole, the proximal ends of the inner sheath tube and the outer sheath tube are inserted from a distal end of corresponding axial sleeve and are fixed, the axial sleeve is provided with a lateral line hole corresponding to the branch portion, the hemostatic valve is arranged at the axial sleeve adjacent to a proximal end thereof and is provided with an elastically contractible hemostatic channel, and the proximal fixing cover is fixed to the proximal end of the axial sleeve and is provided with a pipeline through hole.

Further, the hemostatic valve includes at least one valve plate set, each valve plate set includes a valve plate sleeve and a first valve plate, a second valve plate, and a third valve plate that are arranged coaxially in sequence from a proximal end to a distal end, a first hole is formed in a middle portion of the first valve plate, and non-penetrating notches are formed on two sides of the first valve plate, the non-penetrating notches on the two sides are staggered, a middle portion of the second valve plate protrudes to the distal end and is provide with a second hole at a central position thereof, an annular step is formed on a side of the second valve plate adjacent to the third valve plate, the third valve plate includes an elastic cylinder and two elastic flaps, the elastic flaps are connected to an inner wall of the elastic cylinder, distal ends of the elastic flaps form a flap opening that is openable, the distal ends of the elastic flaps and the inner wall of the elastic cylinder are provided with elastic support ribs configured to elastically close the flap opening, the annular step is embedded in a proximal opening of the elastic cylinder, the valve plate sleeve is a cylindrical member, the elastic cylinder is partially or wholly embedded in the valve plate sleeve, and the first hole, the second hole, and the flap opening form the hemostatic channel.

Further, the branch portion includes a branch main body, a bending adjustment sliding block, and a bending adjustment sleeve, the second stroke assembly includes a second stroke sleeve and a second stroke indicating member, the branch main body includes an axial fixing tube and a guide tube that are in communication with each other, the axial fixing tube is coaxially fixed to an outer side of the axial sleeve, the guide tube is arranged corresponding to the lateral line hole, the guide tube extends towards the proximal end and forms a predetermined acute angle with the axial fixing tube, an interior of the guide tube is provided with a bending adjustment guide slideway along an axial direction thereof, a proximal end of the traction wire is fixed to the bending adjustment sliding block, an inner side of the bending adjustment sleeve drives the bending adjustment sliding block to slide along the bending adjustment guide slideway through threaded fit, the second stroke sleeve is fixedly sleeved on an outer side of the bending adjustment sleeve, the second stroke sleeve is provided with a second stroke slideway, and the outer side of the bending adjustment sleeve drives the second stroke indicating member to slide along the second stroke slideway through threaded fit.

### BRIEF DESCRIPTION OF THE DRAWINGS

FIG. 1 is a schematic structural view of a delivery system for an interventional heart valve stent according to an embodiment of the present application.
FIG. 2 is a schematic view illustrating a connection between the embodiment of the present application and a heart valve stent.
FIG. 3 is a schematic structural view of a securing member according to an embodiment of the present application.
FIG. 4 is a schematic structural view of a securing block according to an embodiment of the present application.
FIG. 5 is a schematic view of a relationship between a guide member, the securing block, and a capsule chamber according to an embodiment of the present application.
FIG. 6 is a schematic structural view of the guide member according to an embodiment of the present application.
FIG. 7 is a schematic structural view of the capsule chamber according to an embodiment of the present application.
FIG. 8 is a schematic view of an external structure of a control handle according to an embodiment of the present application.
FIG. 9 is a schematic cross-sectional view of the control handle according to an embodiment of the present application.
FIG. 10 is a schematic view of an external structure of a bending adjustment handle according to an embodiment of the present application.
FIG. 11 is a schematic view of an internal structure of the bending adjustment handle according to an embodiment of the present application.
FIG. 12 is a schematic view of a hemostatic valve according to an embodiment of the present application. and
FIG. 13 is a schematic structural view of a third valve plate according to an embodiment of the present application.

### DETAILED DESCRIPTION

For easy understanding of the present application, a more comprehensive description of the present application is given below with reference to the accompanying drawings. Preferred embodiments of the present application are given in the accompanying drawings. However, the present application may be implemented in many different forms and is not limited to the embodiments described herein. Rather, these embodiments are provided to make the contents disclosed in the present application more thoroughly and fully understood.

Unless otherwise defined, all technical and scientific terms used herein have the same meaning as commonly understood by those skilled in the art to which the present application belongs. The terms used in the specification of the present application are intended only to describe particular embodiments and are not intended to limit the present application.

An embodiment of the present application provides a delivery system for an interventional heart valve stent, which is mainly configured to deliver the heart valve stent to a patient's heart from outside a body, and includes a delivery tube assembly and a stent securing assembly.

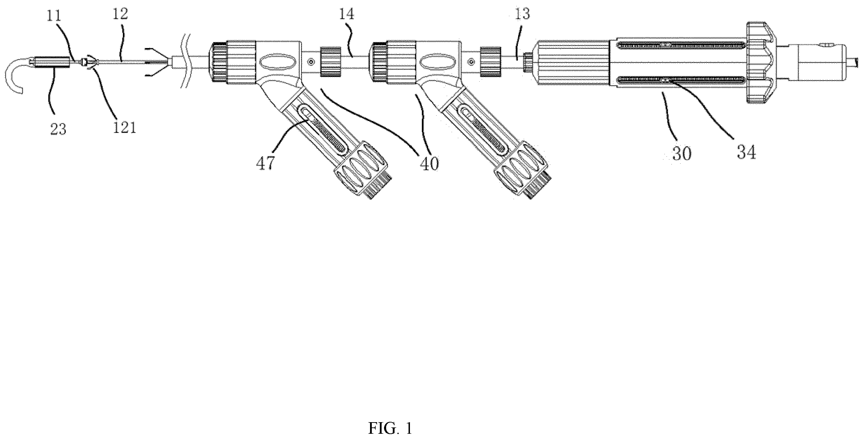

The delivery tube assembly serves as a basic component of the delivery system, which, as shown in FIG. 1, includes an inner core 11, a support tube 12, an inner sheath tube 13, and an outer sheath tube 14 that are sequentially and movably sleeved from inside to outside. Two ends of the inner core 11 extend out of two ends of the support tube 12, respectively. A distal end of the inner sheath tube 13 is provided with an accommodating cavity.

It should be understood that in the present application and embodiments thereof, a proximal end referred to is an end of the delivery system closer to a user in use, and the distal end is an end closer to a patient.

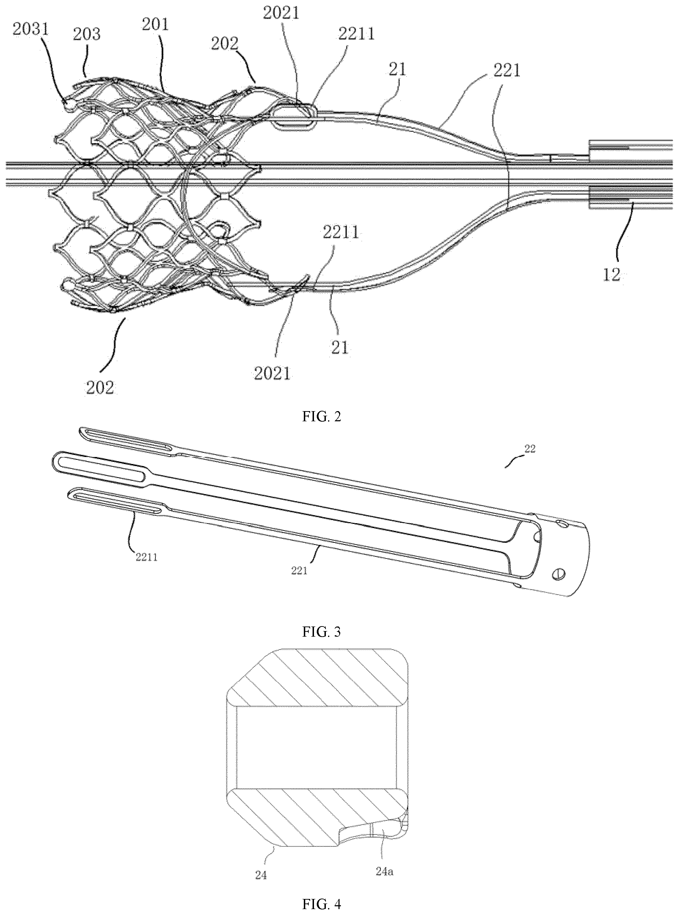

The stent securing assembly is capable of being detachably connected to two ends of a heart valve stent 200 (see FIG. 2), so as to position the heart valve stent at a certain part of the stent securing assembly, generally a distal part. The stent securing assembly includes a capsule chamber 23, a distal end of the inner core 11 extends into the capsule chamber 23 from an opening of a proximal end of the capsule chamber 23 and is fixedly connected to the capsule chamber 23, and the support tube 12 is capable of driving the heart valve stent 200 to move axially to enable the heart valve stent 200 to move in and out of the capsule chamber 23 or from the accommodating cavity.

An inner tube (not shown) is provided between the support tube 12 and the inner sheath tube 13, with a main function of supporting so that the support tube 12 moves in the inner tube. The distal end of the capsule chamber 23 is provided with a TIP head. The TIP head forms a pigtail shape in a natural state.

As a connection manner of the heart valve stent 200 in the present application, the stent securing assembly further includes a limiting member 21, a securing member 22, and a securing block 24.

The securing member 22 includes a support arm 221. The support arm 221 is connected to the support tube 12 and extends towards a distal end, and is configured to be detachably connected to a proximal end of the heart valve stent 200. The securing block 24 is connected to a distal end of the support tube 12 and is configured to be detachably connected to a distal end of the heart valve stent 200.

The limiting member 21 has an elongated strip structure. The limiting member 21 axially extends through the support tube 12 and can extend out of the two ends of the support tube 12. A distal end of the limiting member 21 is configured to connect and limit the proximal end of the heart valve stent 200 and the support arm 221, so as to prevent the proximal end of the heart valve stent 200 and the support arm 221 from being separated from each other.

FIG. 2 is a schematic view of a heart valve stent 200, which includes a stent body 201. The stent body 201 generally has an elastically compressible mesh structure, and has a first release member 202 at a proximal end thereof and a second release member 203 at a distal end thereof.

As an optional embodiment, referring to FIG. 3 and FIG. 4, an end portion of the support arm 221 has a limiting ring 2211. The first release member has a coil 2021 that can extend through the limiting ring 2211 from outside to inside. A distal end of the limiting member 21 can extend to an interior of the coil 2021 to clamp a connection between the coil 2021 and the limiting ring 2211, thereby preventing the coil 2021 and the limiting ring 2211 from separating from each other.

As a preferred embodiment, the securing member 22 has a plurality of support arms 221. The number of the first release member 202 is equal to the number of the support arms 221. The distal end of the limiting member 21 can extend through a plurality of coils 2021 in sequence. For example, in a specific usage condition, the number of the support arms 22 and the number of the second release member are both three. The distal end of the limiting member 21 extends through the three coils 2021 in sequence. When a proximal end of the stent body 201 needs to be released, the limiting member 21 is pulled out from the three coils 2021, and the coils 2021 will be separated from the limiting ring 2211 under tension to complete release of the proximal end of the stent.

As another optional embodiment, the number of the support arm 221 and the number of the first release members 202 are both three, the number of the limiting members 21 is also three, the distal ends of the three limiting members 21 extend through the three coils 2021 in one-to-one correspondence. When the proximal end of the stent body 201 needs to be released, the three limiting members 21 are pulled out from the three coils, respectively, the three first release members 202 are released at the same time, which ensures the quick and smooth release of the bracket body 201.

As a detachable connection mode for the distal end, the securing block 24 is provided with a limiting groove 24a. The first release member 203 has a T-shaped portion 2031 adapted to the limiting groove 24a.

It should be understood that, in some embodiments, the limiting groove 24a may be an opening groove arranged along a radial direction of the stent body 201. In this case, the T-shaped portion 2031 is directly engaged in a notch of the opening groove from an outer portion of the securing block 24, and a stable connection between the limiting groove 24a and the T-shaped portion 2031 is ensured through the pressure of an inner wall of the accommodating cavity or the capsule chamber. When released, the inner wall of the accommodating cavity or the capsule chamber 23 no longer covers an opening of the limiting groove 24a, and under the action of the self-expansion tension, the T-shaped portion 2031 opens outwards along with the stent body 201 and is separated from the limiting groove 24a. In some other embodiments, the limiting groove 24a may alternatively be a slot arranged along an axial direction, and an opening of the slot is arranged towards the proximal end. In this case, the T-shaped portion is inserted into the slot from an end face of the proximal end of the securing block, and the T-shaped portion is stably inserted into the slot by the pressure of the inner wall of the accommodating cavity or the capsule chamber and the tendency of the T-shaped portion to extend towards the distal end, When released, the proximal end of the stent body 201 has a tendency to stretch under the self-expansion tension, so that the T-shaped portion is pulled out of the slot and separated from the limiting groove. The former solution is preferred in the present application.

Similarly, the above detachable connection scheme may alternatively be that the securing block 24 is provided with a limiting protrusion. The first release member is provided with an annular portion detachably connected to the limiting protrusion. The detachable connection between the securing block 24 and the first release member 203 is achieved through sleeving between the annular portion and the limiting protrusion, which will not be described in detail herein.

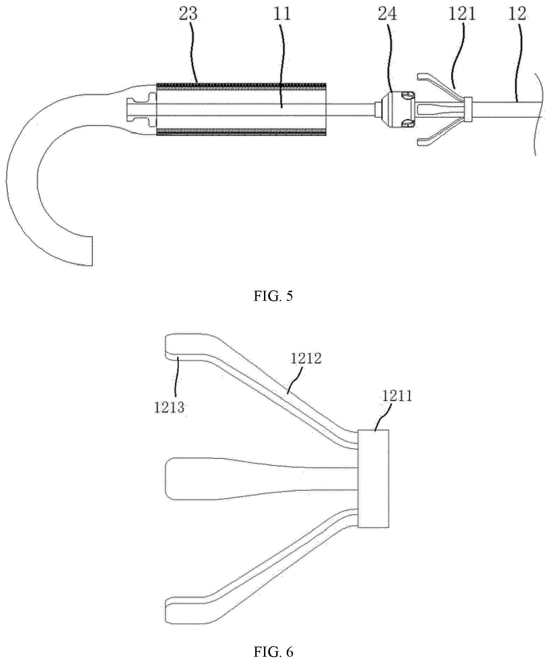

In the embodiment of the present application, as shown in FIG. 5 and FIG. 6, a diameter of an opening of the capsule chamber 23 is capable of being radially expanded. The support tube 12 is provided with a guide member 121 adjacent to the distal end thereof, and the guide member 121 can guide the capsule chamber 23 to move into the outer sheath tube.

After the artificial valve is released, during retraction, if the capsule chamber 23 is not retracted into the outer sheath tube 14, since the capsule chamber 23 is generally made of stainless steel, which is hard, only the capsule chamber 23 made of hard material can withstand the tension of the artificial valve. However, when retracting, the capsule chamber 23 easily scratches a blood vessel wall in a curved blood vessel. After the artificial valve is released at the heart valve of a human body by using the delivery device of the present application, there is a certain distance between the capsule chamber 23 and the outer sheath tube 14, with a certain curvature. After relative movement, an opening of the capsule chamber 23 and an opening of the outer sheath tube 14 are easily misaligned, so that the capsule chamber 23 cannot enter the outer sheath tube 14. If not retracted, since the capsule chamber 23 is harder than the outer sheath tube 14, it is easy to scratch a blood vessel wall when encountering a curved blood vessel during retraction. When the opening of the capsule chamber 23 is in a shape of a bell-mouth, the risk of scratching the blood vessel wall is greater. Therefore, it is necessary to retract the capsule chamber 23 into the outer sheath tube 14. In order to achieve more stable retraction, the guide member 121 is provided on the support tube 22 to form a guide for the capsule chamber 23 to be retracted into the outer sheath tube 14, so as to ensure smooth retraction of the capsule chamber 23, thereby ensuring that a surgery can proceed smoothly during the release and after the release and ensuring efficiency and stability of the surgery.

Specifically, the guide member 121 includes a connecting portion 1211 and a plurality of guide arms 1212. The connecting portion 1211 is fixedly connected to an outer wall of the support tube 22. The plurality of guide arms 1212 extend towards the distal end and elastically expand outwards. Specifically, in this embodiment, the connecting portion 1211 is an annular structure fixed to an outer wall of the support tube 22, and is connected to six guide arms 1212 that are circumferentially evenly arranged. The six guide arms 1212 form a tapered structure with a large opening at the distal end and a small opening at the proximal end on an outer side of the support tube 22.

An inner side of the guide arm 1212 can elastically compress the opening of the capsule chamber 23 and then wrap the capsule chamber 23 in a shape of a bell-mouth. At the same time, the tapered shape formed by the guide arms 1212 can guide the outer sheath tube 14 to retract the capsule chamber 23, which facilitates retraction of the capsule chamber 23 after the artificial valve is loaded.

A distal end of the guide arm 1212 includes a transition portion 1213. The transition portion 1213 is bent toward a side adjacent to an axis of the support tube 30 relative to the guide arm 1212. On the one hand, the transition portion 1213 is configured to warp a barrier opening portion 52 more easily and retract the barrier opening portion 52 into the outer sheath tube 14. On the other hand, the transition portion 1213 allows the guide member 121 to expand outwards in a smaller size, thereby reducing a risk of scratching heart tissue.

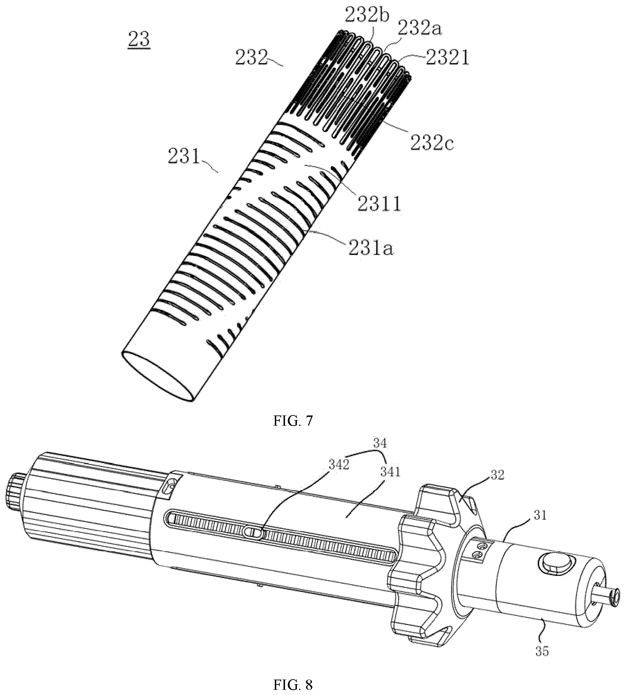

In order to realize that the diameter of the opening of the capsule chamber 23 can be expanded, in the embodiment of the present application, referring to FIG. 7, the capsule chamber 23 includes a chamber body 231 and a barrier opening portion 232. The chamber body 231 is of a straight cylindrical structure. The barrier opening portion 232 is of a straight cylindrical shape without an external force. When the artificial valve is released from the capsule chamber 23, the artificial valve may slowly open and expand the barrier opening portion 232 to form a bell-mouth shape. During retraction, an inner side of the guide arm 322 can elastically press the barrier opening portion 232 and the barrier opening portion 232 can be retracted into the outer sheath tube 14.

An outer diameter of the barrier opening portion 232 ranges from 5 mm to 9 mm, which can meet smooth insertion of the artificial valve without affecting an in vivo operation during release.

Specifically, the barrier opening portion 232 is provided with a plurality of axial notches 232a, the plurality of axial notches 232a are evenly distributed around a circumferential direction of the capsule chamber 23. A portion between the adjacent axial notches 232a form a barrier strip 2321. The barrier strip 2321 make it easy for an open end of the capsule chamber 23 to become a bell-mouth state during the release of the stent.

In order to ensure that the barrier opening portion 232 has better deformation performance, the barrier opening portion 232 is further provided with a first strip hole 232b and a second strip hole 232c. The first strip hole 232b is arranged corresponding to the barrier strip 2321. A length of the first strip hole 232b is greater than a depth of the axial notch 232a. The second strip hole 232c is located between two adjacent first strip holes 232b, and the second bar-shaped holes 232b and the axial notches 232a are spaced apart in the axial direction.

Preferably, in this embodiment, a side surface of the chamber body 231 is provided with a plurality of curved notches 231a distributed along an axial direction of the chamber body 231. Portions between gaps between two ends of all the curved notches 231a are connected to form a connecting rib 2311. The arrangement of the curved notches 231a allows the chamber body 231 to have certain flexibility, thereby adapting to the complex and varied vascular structures inside the human body.

In a more preferred embodiment, all the curved notches 231a have a same length, adjacent curved notches 231a are staggered, and the connecting rib 2311 is spiral along the axial direction. Compared with a linear shape, the spiral shape of connecting rib 2311 can realize universal bending of the chamber body 231.

The chamber body 231 is provided with the curved notch 231a, and the artificial valve needs to expand along the radial direction during the release, which is perpendicular to a direction of the curved notch 231a. Therefore, during release and movement of the artificial valve, resistance may increase due to existence of the curved notch 231a. Therefore, in some more preferred embodiments of the present application, an inner side wall of the chamber body 231 is provided with a first film protective layer to reduce resistance of the inner side wall of the chamber body 231 to the artificial valve. Outer sides of the chamber body 231 and the barrier opening portion 232 are covered with a second film protective layer. The first film protective layer and the second film protective layer are elastic films. Under elastic restoring forces of the first film protective layer and the second film protective layer, the barrier opening portion 232 is retracted from the bell-mouth shape and may even become a straight cylindrical shape.

In some other embodiments of the present application, the barrier opening portion 232 may be made of memory alloy, such as nickel-titanium alloy, so that the barrier opening portion 232 can be retracted from the bell-mouth shape, or even become a straight cylindrical shape.

The above two embodiments in which retraction can be performed further limit the barrier opening portion 232, so that the barrier opening portion 232 can automatically retract after expansion, in order to be more convenient and safer to be retracted into the outer sheath tube 14. Since in actual operation, the size of the barrier opening portion 232 varies after the artificial valve is released, that is, in order to make the system more stable, an opened size of the guide member 121 has to accommodate a maximum size of the barrier opening portion 232. At the heart valve, the larger the guide member 121 is opened, the easier it is to scratch the heart tissue. Therefore, if the barrier opening portion 232 can be retracted under its own restoring force after expansion, the size of the guide member can be reduced, and it can also be more conveniently and safely received into the outer sheath tube 14.

In order to control the delivery system at the proximal end, as shown in FIG. 1, the delivery system further includes a control handle 30 and two bending adjustment handles 40.

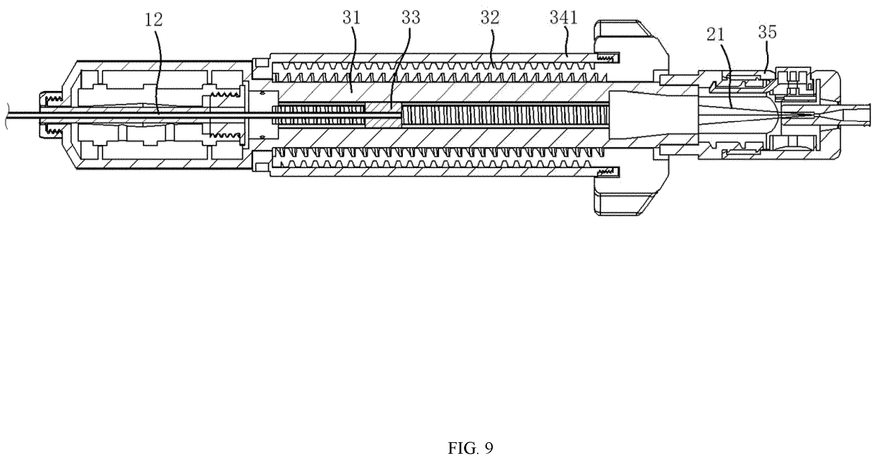

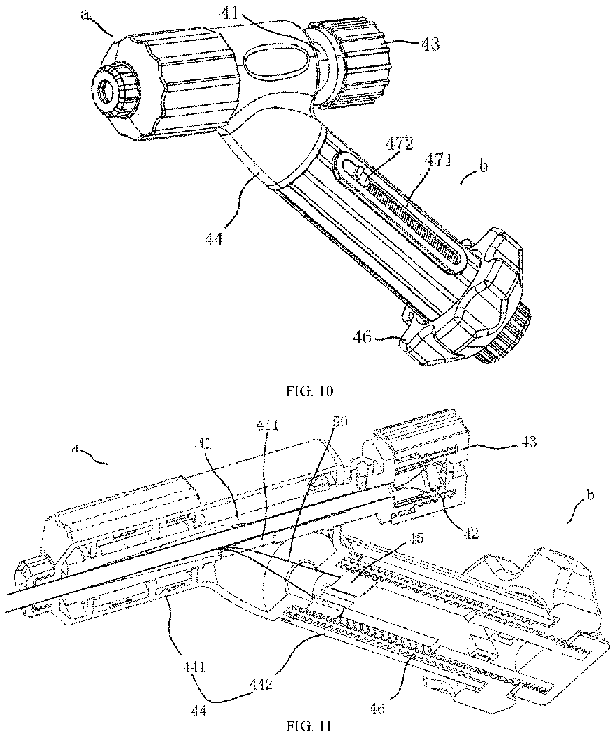

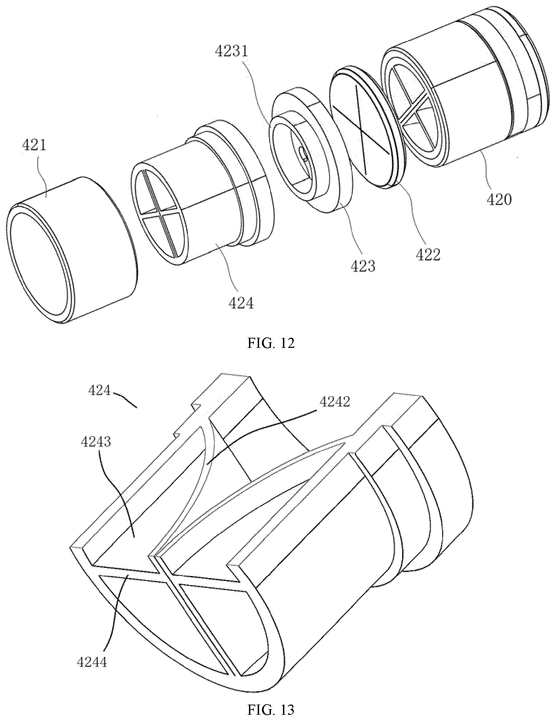

Referring to FIG. 8 to FIG. 13, the control handle 30 is connected to the proximal end of the support tube 12 and can drive the support tube 12 to move axially, thereby driving the capsule chamber 23 to move. The control handle 30 includes a first stroke assembly 34 configured to control an axial movement distance of the support tube 11.

The control handle 30 is arranged along the axial direction of the inner core 11. In an embodiment of the present application, the control handle 30 includes a handle main body sleeve 31, a control sleeve 32, and a control sliding block 33. The first stroke assembly 34 includes a first stroke sleeve 341 and a first stroke indicating member 342.

The handle main body sleeve 31 is provided with an axial control slideway. An inner side of the control sleeve 32 drives the control sliding block 33 to slide along the axial control slideway through threaded fit. As an optional connection manner, the handle main body sleeve 31 is of a cylindrical structure, The control sleeve 32 is sleeved on the outer side surface of the handle main body sleeve 31 and can rotate coaxially outside the handle main body sleeve 31. The inner side of the control sleeve 32 has internal threads. Both sides of the axial control slideway are provided with guide grooves. The control sliding block 33 is axially mounted on the axial control slideway and an outer side thereof protrudes from the guide groove to fit with the internal threads of the control sleeve 32 When the control sleeve 32 rotates, the control sliding block 33 is driven to slide axially.

The proximal end of the support tube 12 is fixed to the distal end of the handle main body sleeve 21 by a compression bolt. The support tube 11 extends into the distal end of the handle main body sleeve 21 and is fixed to the control sliding block 23, so that the axial sliding of the control sliding block 23 can drive the support tube 12 to move axially.

The first stroke sleeve 341 is coaxially and fixedly sleeved on the outer side of the control sleeve 32 The first stroke sleeve 341 is provided with a first stroke slideway. The outer side of the control sleeve 32 has external threads. The first stroke indicating member 342 fits with the external threads of the control sleeve 32. When the control sleeve 32 rotates, the first stroke indicating member 342 slides on the first stroke slideway. The specifications of the threads on two sides of the control sleeve 32 are set to be the same or preset as prescribed. A stroke scale configured to indicate a moving distance is provided on the first stroke slideway, so that a sliding distance of the control sliding block 23 can be known through a sliding distance of the first stroke indicating member 342 on the first stroke slideway, thereby accurately controlling the axial movement distance of the support tube 12. Moreover, in the present application, by thread driving, adjustment accuracy thereof is high, stopping and locking at any time throughout the process can be realized, and the release process of the valve stent can be accurately monitored to reduce difficulty of release of the stent.

In order to facilitate the control of the limiting member 21 at the proximal end, the control handle 30 further includes a dismounting member 35 detachably mounted at a proximal end of the handle main body sleeve 31 and connected to a proximal end of the limiting member 21. When the heart valve stent 200 is located in the capsule chamber 23, as mentioned above, the limiting member 21 can be pulled by the dismounting member 35, so that the distal end of the limiting member 21 releases the connection and limitation between the proximal end of the heart valve stent 200 and the support arm 221.

The two bending adjustment handles 40 are provided at a proximal end of the inner sheath tube 13 and a proximal end of the outer sheath tube 14, respectively. Corresponding distal ends of the inner sheath tube 13 and the outer sheath tube 14 are bent in the same plane or different planes through a traction wire 50. The bending adjustment handles 40 each includes a second stroke assembly 47 configured to control a bending curvature of corresponding inner sheath tube 13 or the outer sheath tube 14.

It should be noted that most of the blood vessels in the human body are bent in a three-dimensional direction. In some surgical operations, a sheath is generally required to extend through an aortic arch after entering through a femoral artery, and the blood vessel here has a large bending angle and bends in the three-dimensional direction, which has high requirements for delivery of the heart valve stent. If only one bending sheath that may be bent in one plane is used, when entering the aortic arch, the sheath first bends in one plane and then bends to another plane through friction and guidance with the blood vascular wall. This manner also allows entry to the heart. However, if it is bent by large friction and guidance with the blood vessel, it is easy to cause damage to the blood vessel wall, the operation time will increase a lot, and there will be a lot of risks. Secondly, in interventional surgery, a doctor operates the control handle, and the sheath moves in the human body, which can only be displayed on a device through a development of the sheath. That is, the blood vessel wall cannot be seen on the device, only the sheath can be seen. Therefore, how the sheath should be bent next can only be estimated by the bending degree of the sheath. Then, in the case of stroke control, the bending degree of the sheath can be directly fed back to the stroke control curvature, so that how much curvature the sheath needs to continue to bend can also be accurately performed through the stroke control. Therefore, in summary, when the sheath enters the aortic arch that has a large bending angle and bends in the three-dimensional direction, the double-layer bending adjustment and stroke control are very necessary, so that the sheath can quickly reach the heart through the aortic arch and the time is saved.

In the embodiment of the present application, the two bending adjustment handles 40 are sequentially arranged on a side of the control handle 30 adjacent to a distal end of an inner tube 10, and the bending adjustment handle 40 configured to adjust the inner sheath tube 13 is arranged closer to the control handle 20, so that the inner sheath tube 13 and the outer sheath tube 14 are bent, respectively.

Specifically, the bending adjustment handle 40 includes an axial portion a and a branch portion b, the axial portion a is arranged along an axial direction of the inner tube 10, the proximal ends of the inner sheath tube 13 and the outer sheath tube 14 are fixed to the axial portions of the corresponding bending adjustment handles 40, and the branch portion b forms an acute angle with the axial portion a. The axial portion a and the branch portion b form a certain angle, which effectively shortens an overall length of the delivery system. The second stroke assembly is arranged on the branch portion b. The bending of the branch portion b and the bending of the corresponding sheath belong to the same bending arc, so as to have an effect of guiding the bending. Moreover, on the second stroke assembly, a linear stroke is transformed into bending curvature more smoothly, there will be no large bending action within a stroke, thereby the stability of the device.

The axial portion a includes an axial sleeve 41, a hemostatic valve 42 and a proximal fixing cover 43. The axial sleeve 41 is provided with an axial through hole. The proximal ends of the inner sheath tube 13 and the outer sheath tube 14 are inserted from the distal end of the corresponding axial sleeve 41 and are locked. The axial sleeve 41 is provided with a lateral line hole 411 corresponding to the branch portion b. The hemostatic valve 42 is arranged at the axial sleeve 41 adjacent to a proximal end thereof and is provided with an elastically contractible hemostatic channel. The proximal fixing cover 43 is fixed to the proximal end of the axial sleeve 41 and is provided with a pipeline through hole.

The hemostatic valve 42 is mainly configured to prevent blood from leaking through the sheath under blood pressure when the sheath enters the blood vessel. In the embodiment of the present application, the hemostatic valve 42 includes at least one valve plate set 420. In a preferred embodiment of the present application, the hemostatic valve 42 includes two valve plate sets 420 arranged in the axial direction.

The valve plate set 420 includes a valve plate sleeve 421 and a first valve plate 422, a second valve plate 423, and a third valve plate 424 that are coaxially s arranged in sequence from the proximal end to the distal end. A first hole is formed in a middle portion of the first valve plate 422. Non-penetrating notches are formed on two sides of the first valve plate 422, and the non-penetrating notches on the two sides are staggered. In the present application, cross grooves are formed on two sides of the first valve plate 422, the two cross grooves are staggered by 45 degrees, and the middle portion of the first valve plate 422 can be effectively axially raised towards the two sides. The middle portion of the second valve plate 423 protrudes to the distal end and is provided with a second hole at a central position thereof. An annular step 4231 is formed on a side of the second valve plate 423 adjacent to the third valve plate 424. The third valve plate 424 includes an elastic cylinder 4241 and two elastic flaps 4242. The elastic flaps 4242 are connected to an inner wall of the elastic cylinder 4241, Distal ends of the two elastic flaps 4242 form a flap opening that can be opened and closed. The distal ends of the elastic flaps 4242 and the inner wall of the elastic cylinder 4241 are provided with elastic support ribs 4243 configured to elastically close the flap opening. The annular step 4231 is embedded in a proximal opening of the elastic cylinder 4241. The valve plate sleeve 421 is a cylindrical member. The elastic cylinder 4241 is partially or wholly embedded in the valve plate sleeve 421. The first hole, the second hole and the flap opening form the hemostatic channel. Through the above structural arrangement, when the valve plate set 420 moves relative to the inner tube 10 or the inner sheath tube 13, middle portions of the second valve plate 423 and the third valve plate 424 can protrude axially through friction, which can effectively prevent blood leakage.

In the embodiment of the present application, the branch portion b includes a branch main body 44, a bending adjustment sliding block 45, and a bending adjustment sleeve 46. The second stroke assembly 47 includes a second stroke sleeve 471 and a second stroke indicating member 472. The branch main body 44 includes an axial fixing tube 441 and a guide tube 442 that are in communication with each other. The axial fixing tube 441 is coaxially fixed to an outer side of the axial sleeve 41. The guide tube 442 is arranged corresponding to the lateral line hole. The guide tube 442 extends towards the proximal end and forms a predetermined acute angle with the axial fixing tube 441An interior of the guide tube 442 is provided with a bending adjustment guide slideway along an axial direction thereof. A proximal end of the traction wire 50 is fixed to the bending adjustment sliding block 45. The principle of bending adjustment is that the bending adjustment sliding block 45 is driven by the bending adjustment sleeve 46 to move in a straight line and then the proximal end of the traction wire is moved to pull the distal end of the corresponding inner sheath tube 13 or outer sheath tube 14 to bend. The connection between the bending adjustment sliding block 45 and the bending adjustment sleeve 46 is similar to that between the control sleeve 32 and the control sliding block 33. That is, an inner side of the bending adjustment sleeve 46 drives the bending adjustment sliding block 45 to slide along the bending adjustment guide slideway through threaded fit.

The second stroke sleeve 471 is fixedly sleeved on the outer side of the bending adjustment sleeve 46. The second stroke sleeve 471 is provided with a second stroke slideway. The outer side of the bending adjustment sleeve 46 drives the second stroke indicating member 472 to slide along the second stroke slideway through threaded fit.

The second stroke indicating member 472 may make judgment according to an observed movement distance or a movement thread distance, or a specific stroke scale may be provided on a surface of the second stroke sleeve 471 for judgment, so as to adjust a proximal motion distance of the traction wire, thereby achieving accurate control of bending curvature of the inner sheath tube 13 or the outer sheath tube 14.

According to an aortic valve delivery system in the present application, the support tube 12 is driven by the control handle 30 to move axially. The two bending adjustment handles 40 drive the distal ends of the inner sheath tube 13 and the outer sheath tube 14 to bend through the corresponding traction wire 50. The control handle 20 is provided with the first stroke assembly 24 to accurately control the axial movement distance of the support tube 11, and the bending adjustment handle 40 is provided with the second stroke assembly 47 to control bending curvature of the sheath, thereby bettering controlling bending and release of the artificial valve.

The above are merely preferred embodiments of the present disclosure, but are not intended to limit the present disclosure. Any amendment, equivalent replacement, improvement, and the like made within the spirit and principle of the present disclosure should fall within the protection scope of the present disclosure.

## Claims

1. A delivery system for an interventional heart valve stent, comprising:
a delivery tube assembly comprising an inner core, a support tube, an inner sheath tube, and an outer sheath tube that are sequentially and movably sleeved from inside to outside, wherein two ends of the inner core extend out of two ends of the support tube, respectively, and a distal end of the inner sheath tube is provided with an accommodating cavity; and
a stent securing assembly capable of being detachably connected to two ends of a heart valve stent, the stent securing assembly comprising a capsule chamber, wherein a distal end of the inner core extends into the capsule chamber from an opening of a proximal end of the capsule chamber and is fixedly connected to the capsule chamber, and the support tube is capable of driving the heart valve stent to move axially to enable the heart valve stent to move in and out of the capsule chamber or from the accommodating cavity.

2. The delivery system for an interventional heart valve stent according to claim 1, wherein the stent securing assembly further comprises a securing member, a securing block, and a limiting member;
the securing member comprises a support arm connected to the support tube and extending towards a distal end, the securing block is connected to a distal end of the support tube, and the support arm and the securing block are respectively configured to be detachably connected to a proximal end and a distal end of the heart valve stent; and
the limiting member has an elongated strip structure, the limiting member axially extend through the support tube and is capable of extending out of the two ends of the support tube, and a distal end of the limiting member is configured to connect and limit the proximal end of the heart valve stent and the support arm, so as to prevent the proximal end of the heart valve stent and the support arm from being separated from each other.

3. The delivery system for an interventional heart valve stent according to claim 1, wherein a diameter of an opening of the capsule chamber is capable of being radially expanded, the support tube is provided with a guide member adjacent to the distal end thereof, and the guide member is capable of guiding the capsule chamber to move into the outer sheath tube.

4. The delivery system for an interventional heart valve stent according to claim 3, wherein the guide member comprises a connecting portion and a plurality of guide arms, the connecting portion is fixedly connected to an outer wall of the support tube, and the plurality of guide arms extend towards a distal end and elastically expand outwards.

5. The delivery system for an interventional heart valve stent according to claim 4, wherein a distal end of the guide arm comprises a transition portion, the transition portion is bent towards a side adjacent to an axis of the support tube relative to the guide arm.

6. The delivery system for an interventional heart valve stent according to claim 3, wherein the capsule chamber comprises a chamber body and a barrier opening portion, the chamber body is of a straight cylindrical structure, the barrier opening portion is of in a straight cylindrical shape without an external force and is capable of being expanded into a bell-mouth shape under an external force, and an inner side of the guide arm is capable of elastically pressing the barrier opening portion.

7. The delivery system for an interventional heart valve stent according to claim 6, wherein the barrier opening portion is provided with a plurality of axial notches, the plurality of axial notches are evenly distributed around a circumferential direction of the capsule chamber, and a portion between adjacent axial notches form a barrier strip.

8. The delivery system for an interventional heart valve stent according to claim 6, wherein a side surface of the chamber body is provided with a plurality of curved notches with a same length, the plurality of curved notches are distributed along an axial direction of the chamber body, adjacent curved notches are staggered, and portions between gaps between two ends of curved notches are connected to form a connecting rib that is spiral along the axial direction.

9. The delivery system for an interventional heart valve stent according to claim 1, further comprising a control handle and two bending adjustment handles, wherein the control handle is connected to a proximal end of the support tube and is capable of driving the support tube to move axially, the control handle comprises a first stroke assembly configured to control an axial movement distance of the support tube, the two bending adjustment handles are provided at a proximal end of the inner sheath tube and a proximal end of the outer sheath tube, respectively, corresponding distal ends of the inner sheath tube and the outer sheath tube are bent in a same plane or different planes through a traction wire, and the bending adjustment handles each comprises a second stroke assembly configured to control a bending curvature of the corresponding inner sheath tube or the outer sheath tube.

10. The delivery system for an interventional heart valve stent according to claim 9, wherein the control handle is arranged along an axial direction of the support tube, the two bending adjustment handles are sequentially arranged on a side of the control handle adjacent to a distal end of the support tube, the bending adjustment handle configured to adjust the inner sheath tube is arranged closer to the control handle, the bending adjustment handle comprises an axial portion and a branch portion, the axial portion is arranged along the axial direction of the support tube, the proximal ends of the inner sheath tube and the outer sheath tube are fixed to the axial portions of the corresponding bending adjustment handles, the branch portion forms an acute angle with the axial portion, and the second stroke assembly is arranged on the branch portion.

11. The delivery system for an interventional heart valve stent according to claim 10, wherein the control handle comprises a handle main body sleeve, a control sleeve, and a control sliding block, the first stroke assembly comprises a first stroke sleeve and a first stroke indicating member, the handle main body sleeve is provided with an axial control slideway, an inner side of the control sleeve drives the control sliding block to slide along the axial control slideway through threaded fit, the support tube is fixed to a distal end of the handle main body sleeve, the support tube extends into the distal end of the handle main body sleeve and is fixed to the control sliding block, the first stroke sleeve is fixedly sleeved on an outer side of the control sleeve, the first stroke sleeve is provided with a first stroke slideway arranged along an axial direction thereof, and the outer side of the control sleeve drives the first stroke indicating member to slide along the first stroke slideway through threaded fit.

12. The delivery system for an interventional heart valve stent according to claim 11, wherein the control handle further comprises a dismounting member detachably mounted at a proximal end of the handle main body sleeve and connected to a proximal end of the limiting member, and when the heart valve stent is located in the capsule chamber, the limiting member is pulled by the dismounting member, so that a distal end of the limiting member releases a connection and limitation between a proximal end of the heart valve stent and the support arm.

13. The delivery system for an interventional heart valve stent according to claim 11, wherein the axial portion comprises an axial sleeve, a hemostatic valve, and a proximal fixing cover, the axial sleeve is provided with an axial through hole, the proximal ends of the inner sheath tube and the outer sheath tube are inserted from a distal end of corresponding axial sleeve and are fixed, the axial sleeve is provided with a lateral line hole corresponding to the branch portion, the hemostatic valve is arranged at the axial sleeve adjacent to a proximal end thereof and is provided with an elastically contractible hemostatic channel, and the proximal fixing cover is fixed to the proximal end of the axial sleeve and is provided with a pipeline through hole.

14. The delivery system for an interventional heart valve stent according to claim 13, wherein the hemostatic valve comprises at least one valve plate set, each valve plate set comprises a valve plate sleeve and a first valve plate, a second valve plate, and a third valve plate that are arranged coaxially in sequence from a proximal end to a distal end, a first hole is formed in a middle portion of the first valve plate, and non-penetrating notches are formed on two sides of the first valve plate, the non-penetrating notches on the two sides are staggered, a middle portion of the second valve plate protrudes to the distal end and is provide with a second hole at a central position thereof, an annular step is formed on a side of the second valve plate adjacent to the third valve plate, the third valve plate comprises an elastic cylinder and two elastic flaps, the elastic flaps are connected to an inner wall of the elastic cylinder, distal ends of the elastic flaps form a flap opening that is openable, the distal ends of the elastic flaps and the inner wall of the elastic cylinder are provided with elastic support ribs configured to elastically close the flap opening, the annular step is embedded in a proximal opening of the elastic cylinder, the valve plate sleeve is a cylindrical member, the elastic cylinder is partially or wholly embedded in the valve plate sleeve, and the first hole, the second hole, and the flap opening form the hemostatic channel.

15. The delivery system for an interventional heart valve stent according to claim 13, wherein the branch portion comprises a branch main body, a bending adjustment sliding block, and a bending adjustment sleeve, the second stroke assembly comprises a second stroke sleeve and a second stroke indicating member, the branch main body comprises an axial fixing tube and a guide tube that are in communication with each other, the axial fixing tube is coaxially fixed to an outer side of the axial sleeve, the guide tube is arranged corresponding to the lateral line hole, the guide tube extends towards the proximal end and forms a predetermined acute angle with the axial fixing tube, an interior of the guide tube is provided with a bending adjustment guide slideway along an axial direction thereof, a proximal end of the traction wire is fixed to the bending adjustment sliding block, an inner side of the bending adjustment sleeve drives the bending adjustment sliding block to slide along the bending adjustment guide slideway through threaded fit, the second stroke sleeve is fixedly sleeved on an outer side of the bending adjustment sleeve, the second stroke sleeve is provided with a second stroke slideway, and the outer side of the bending adjustment sleeve drives the second stroke indicating member to slide along the second stroke slideway through threaded fit.
